# EUROPÄISCHE PATENTSCHRIFT

(11) **EP 0 846 753 B1**
(45) Veröffentlichungstag und Bekanntmachung des Hinweises auf die Patenterteilung: **16.10.2002**
(21) Anmeldenummer: 97122735.0
(22) Anmeldetag: 07.06.1994
(51) Int. Cl.: C11D 3/33

(54) **Verwendung von alpha-Alanin-N,N-diessigsäure und ihren Salzen als biologisch abbaubare Komplexbildner für Erdalkali und Schwermetallionen in alkalischen Reinigungsformulierungen für Reinigungsprozesse in der Getränke- und Nahrungsmittelindustrie**
Use of alpha-Alanine-N,N-diacetic acid and salts as biodegradable complexing agents for alkaline earth and heavy metal ions in alkali detergent compositions and cleaning process in beverage and food industry
Utilisation de l'acide alpha-Alanine et ses sels comme agent complexant biodegradable pour des ions de métaux lourds et alcalino-terreux dans des compositions détergentes et procédés de lavage en les industries des boissons et denrées alimentaires

(30) Priorität: 16.06.1993 DE 4319935
(43) Veröffentlichungstag der Anmeldung: 10.06.1998
(62) Teilanmeldung aus: 94920434.1
(73) Patentinhaber: BASF AKTIENGESELLSCHAFT, 67056 Ludwigshafen (DE)
(72) Erfinder: Schneider, Juergen, Dr., 67251 Freinsheim (DE); Potthoff-Karl, Birgit, Dr., 67061 Ludwigshafen (DE); Kud, Alexander, Dr., 55234 Eppelsheim (DE); Baur, Richard, Dr., 67112 Mutterstadt (DE); Oftring, Alfred, Dr., 67098 Bad Duerkheim (DE); Greindl, Thomas, Dr., 94127 Neuburg (DE)

(56) Entgegenhaltungen:
- EP-A- 0 030 461
- EP-A- 0 356 974
- US-A- 2 500 019

## Beschreibung

Die vorliegende Erfindung betrifft die Verwendung von α-Alanin-N,N-diessigsäure und ihren Salzen als biologisch abbaubare Komplexbildner für Erdalkali- und Schwermetallionen in alkalischen Reinigungsmittelformulierungen für Reinigungsprozesse in der Getränke- und Nahrungsmittelindustrie.

Aus den japanischen Offenlegungsschriften 80/157 695 (1) und 80/160 099 (2), zitiert in Chem. Abstr. 95 (1981), 9123 m bzw. 9124 n, ist bekannt, Alanin-N,N-diessigsäure in Form des Natriumsalzes als Builder in pulverförmig formulierten Textilwaschmitteln einzusetzen, wobei insbesondere für Baumwolltextilien eine Waschkraftverstärkung zu beobachten ist.

Die EP-A 089 136 (3) betrifft Mundhygieneprodukte, die als Calciumsequestriermittel u.a. α-Alanin-N,N-diessigsäure enthalten. Diese dienen dazu, die Menge an Calciumfluorid, welche dem Zahnschmelz zum Kariesschutz zugeführt wird, zu kontrollieren.

Aus der EP-A 356 974 ist bekannt, daß β-Alanin-N,N-diessigsäure und ihre Salze in Reinigungsmitteln generell eingesetzt werden können.

Als Komplexbildner für Erdalkali- und Schwermetallionen auf den verschiedensten technischen Gebieten mit ihren teilweise stark voneinander abweichenden Anforderungs- und Problemfeldern werden üblicherweise immer noch altbekannte und bewährte Systeme wie Polyphosphate, Nitrilotriessigsäure oder Ethylendiaminintetraessigsäure eingesetzt. Diese Mittel zeigen allerdings gewisse Nachteile, prinzipielle Schwachpunkte sind insbesondere ihr noch verbesserungsbedürftiges Calcium- und Mangan-Bindevermögen, ihre noch nicht optimale stabilisierende Wirkung in Bleichbädern und Bleichsystemen, sowie ihre meist unzureichende biologische Abbaubarkeit bzw. Eliminierbarkeit.

Aufgabe der vorliegenden Erfindung war es daher, Komplexbildner bereitzustellen, welche die Nachteile des Standes der Technik nicht mehr aufweisen.

Demgemäß wurde die Verwendung von α-Alanin-N,N-diessigsäure und ihren Salzen der Formel I 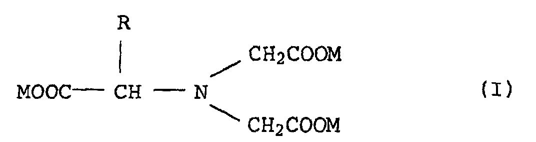 in der R für Methyl steht und
- M: Wasserstoff, Alkalimetall, Ammonium oder substituiertes Ammonium bedeutet,
als Komplexbildner für Erdalkali- und Schwermetallionen in alkalischen Reinigungsmittelformulierungen für Reinigungsprozesse in der Getränke- und Nahrungsmittelindustrie gefunden.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Die erfindungsgemäße Verwendung für α-Alanin-N,N-diessigsäure und ihre Salze I liegt in alkalischen Reinigungsmittelformulierungen für die Getränke- und Nahrungsmittelindustrie, insbesondere für die Flaschenreinigung in der Getränkeindustrie sowie die Apparatereinigung in Molkereien, in Brauereien, in der Konserven-, der Backwaren-, der Zucker-, der fettverarbeitenden und der fleischverarbeitenden Industrie.

Für die Reinigung von Behältnissen und Apparaturen in der Getränke- und Nahrungsmittelindustrie wurden Formulierungen insbesondere mit verbesserten Eigenschaften bei der Schmutzentfernung gesucht. Zur Verringerung der Abwasserbelastung ist es außerdem wünschenswert, ganz auf organische Lösungsmittel in derartigen Formulierungen zu verzichten.

Die vorliegenden alkalischen Reinigungsmittelformulierungen weisen in der Regel pH-Werte von 8 bis 14, vorzugsweise von 9 bis 13, insbesondere von 10 bis 12, auf.

Ein bevorzugtes Einsatzgebiet für die beschriebenen Reinigungsmittelformulierungen ist die Flaschenreinigung in der Getränkeindustrie, insbesondere mit automatischen Flaschenspülmaschinen mit Stundenleistungen bis zu üblicherweise 30.000 bis 70.000 Flaschen. Die verschmutzten Flaschen enthielten beispielsweise Bier, Milch, Erfrischungsgetränke, Fruchtsäfte, Süßmost oder Mineralwasser.

Ein weiteres bevorzugtes Einsatzgebiet für die beschriebenen Reinigungsmittelformulierungen ist die Apparatereinigung in Molkereien. Bei der Reinigung von Butterfertigern, bei der es hauptsächlich auf die Entfettung ankommt, können sie mit vorteilhafter Wirkung eingesetzt werden. Insbesondere jedoch dort, wo Rückstände oder Beläge aus Calciumphosphat, anderen Calciumsalzen zumeist organischer Säuren und Casein ("Milchstein") zu entfernen sind, also z. B. bei Milch-Plattenerhitzern, Tellereinsätzen von Milchzentrifugen oder Lager- und Transport-Tanks für Milch, eignen sich Glycin-N,N-diessigsäure-Derivate I bzw. ihre Salze enthaltende Reinigungsmittel in hervorragender Weise.

Ein weiteres bevorzugtes Einsatzgebiet für die beschriebenen Reinigungsmittelformulierungen ist die Apparatereinigung in Brauereien. Hier sind vor allem Rückstände oder Beläge aus Calciumoxalat, Hopfenharzen und Eiweißverbindungen ("Bierstein") zu entfernen, beispielsweise aus Gärtanks, Lagertanks oder Bierleitungen.

Ein weiteres bevorzugtes Einsatzgebiet für die beschriebenen Reinigungsmittelformulierungen ist die Apparatereinigung in der Konservenindustrie. Beim Erhitzen der mit Nahrungsmitteln gefüllten und geschlossenen Blechdosen, üblicherweise in einem Autoklaven, oder bei der Endreinigung von Dosen, z. B. in einer Durchlaufspritzmaschine, müssen Reinigungsmittel mitverwendet werden, die die Reste des Abfüllgutes abwaschen, ohne das Weißblech oder dessen Lackierung anzugreifen. Außerdem soll das Reinigungsmittel verhindern, daß sich Wassersteinbeläge auf den Dosen oder in den Apparaten abscheiden.

Ein weiteres bevorzugtes Einsatzgebiet für die beschriebenen Reinigungsmittelformulierungen ist die Apparatereinigung in der Backwarenindustrie, insbesondere die Reinigung von Back- und Teigformen, welche mit angebrannten Backfett- und Teigresten verunreinigt sind. Die Reinigung geschieht üblicherweise durch Abkochen mit den alkalischen Reinigungslösungen oder durch Waschen in Durchlaufspritzanlagen.

Ein weiteres bevorzugtes Einsatzgebiet für die beschriebenen Reinigungsmittelformulierungen ist die Apparatereinigung in der Zuckerindustrie. Bei der Gewinnung von Saccharose aus Zuckerrüben oder Zuckerrohr fallen Calciumsalze enthaltende Verunreinigungen oder Rückstände an, für deren Entfernung sich die Verbindung I bzw. ihre Salze enthaltenden beschriebenen Formulierungen in hervorragender Weise eignen.

Ein weiteres bevorzugtes Einsatzgebiet für die beschriebenen Reinigungsmittelformulierungen ist die Apparatereinigung in der fettverarbeitenden Industrie, die aus Fetten tierischen oder pflanzlichen Ursprungs vor allem Schmalz, Talg, Speiseöle oder durch katalytische Hydrierung gehärtete Fette oder fette Öle, z. B. Margarine, erzeugt. Derartige Produkte stellen neben ihrer Bedeutung auf dem Nahrungsmittelbereich auch wichtige Rohstoffe für die Herstellung von Produkten zur Textilveredlung, von Anstrichmitteln, Lederpflegemitteln, kosmetischen Präparaten, Kerzen, Seifen, Tensiden, Schmierstoffen, Weichmachern, Zementund Asphaltzusätzen sowie von Kunststoffen dar.

Ein weiteres bevorzugtes Einsatzgebiet für die beschriebenen Reinigungsmittelformulierungen ist die Apparatereinigung in der fleischverarbeitenden Industrie. Hier müssen insbesondere wassersteinverhütende Reinigungsmittel eingesetzt werden, z. B. in den sog. Dampfstrahl-Reinigungsgeräten, bei denen ein heißes Dampf-Flüssigkeits-Gemisch auf die zu reinigenden Apparate und Geräte gestrahlt wird.

Die beschriebenen α-Alanin-N,N-diessigsäure bzw. ihre Salze I enthaltenden alkalischen Reinigungsmittelformulierungen können weitgehend frei von organischen Lösungsmitteln zum Einsatz gebracht werden. Somit wird eine mögliche Umweltbelastung weitgehend ausgeschlossen.

Eine für die aufgezählten Einsatzgebiete der Getränke- und Nahrungsmittelindustrie übliche wäßrige Reinigungsmittelformulierung enthält
(i) 0,05 bis 30 Gew.-%, vorzugsweise 0,1 bis 25 Gew.-%, insbesondere 0,5 bis 15 Gew.-% α-Alanin-N,N-diessigsäure I oder Alkalimetall-, Ammonium- oder substituierte Ammoniumsalze hiervon,
(ii) 2 bis 50 Gew.-%, vorzugsweise 5 bis 40 Gew.-%, insbesondere 8 bis 25 Gew.-% Alkalimetallhydroxid, -carbonat, -silicat oder einer Mischung hieraus und
(iii) 1 bis 30 Gew.-%, vorzugsweise 2 bis 25 Gew.-%, insbesondere 3 bis 20 Gew.-% Tenside.

Hierbei eignen sich als Komponente (ii) vor allem Natrium- und Kaliumhydroxid, daneben aber auch Natrium- und Kaliumcarbonat; es können auch Mischungen der genannten Alkalien eingesetzt werden.

Als Tenside (iii) können alle üblichen anionischen oder nichtionischen Tenside oder Mischungen hieraus verwendet werden, vor allem eignen sich jedoch Alkylsulfate, Alkylsulfonate, Fettalkoholalkoxylate, Oxoalkoholalkoxylate, Alkylpolyglucoside und Fettaminalkoxylate.

Diese Zusammensetzung stellt eine Grundformulierung für alle genannten Anwendungsgebiete dar. Im einzelnen innerhalb dieser Grundformulierung voneinander abweichende Zusammensetzung sind durch die verschiedenen Arten von Nahrungsmittel- und Getränkeverschmutzungen, die unterschiedlichen Mengen an Erdalkalimetallionen in diesen Rückständen und Belägen sowie durch die unterschiedlich empfindlichen Materialien der zu reinigenden Behältnissen und Apparaturen bei den verschiedenen Anwendungsgebieten zu erklären. In diesem Zusammenhang ist auch erwähnenswert, daß die beschriebenen alkalischen Reinigungsmittelformulierungen, welche α-Alanin-N,N-diessigsäure I oder ihre Salze enthalten, in der Regel keine Korrosionen, auch bei empfindlichen Apparatematerialien, hervorrufen.

Die oben beschriebene Grundformulierung aus den Komponenten (i) bis (iii) kann noch übliche Hilfsmittel in den hierbei üblichen Konzentrationen enthalten, beispielsweise Desinfektionsmittel zur Erzielung des angestrebten bakteriologischen Reinheitsgrades, Netzmittel, Lösevermittler, Korrosionsinhibitoren oder Konservierungsmittel.

α-Alanin-N,N-diessigsäure und ihre Salze I eignen sich vor allem deshalb so gut für den beschriebenen Anwendungszweck, weil sie außerordentlich effektive Komplexbildner für Erdalkalimetallionen und für Schwermetallionen, insbesondere für Calcium, darstellen. Ihr Calciumbindevermögen ist außergewöhnlich hoch.

Weitere Vorteile sind ihr sehr geringes Toxizitätspotential und ihre gute biologische Abbaubarkeit. So zeigt α-Alanin-N,N-diessigsäure im Zahn-Wellens-Test unter Standardbedingungen eine biologische Abbaubarkeit > 90 % (28-Tage-Wert), wogegen beispielsweise Ethylendiamintetraessigsäure unter gleichen Bedingungen einen Wert von < 10 % ergibt.

Im Zusammenhang mit ihrer guten biologischen Abbaubarkeit ist es auch sehr vorteilhaft, daß die die Verbindungen I enthaltenden Reinigungsmittelformulierungen meist weitgehend frei von organischen Lösungsmitteln zum Einsatz gebracht werden können. Somit wird eine mögliche Umweltbelastung noch weitgehender ausgeschlossen.

Gegenstand der vorliegenden Erfindung sind auch alkalische Reinigungsmittelformulierungen für die Getränke- und Nahrungsmittelindustrie, welche wirksame Mengen an α-Alanin-N,N-diessigsäure oder ihren Salzen I enthalten.

Herstellungsbeispiele

### Beispiel 1

### Herstellung von α-D,L-Alanin-N,N-diessigsäure-Trinatriumsalz aus Iminodiacetonitril

Zu einer Suspension von 95 g Iminodiacetonitril (100 gew.-%ig) in 500 ml Wasser wurden nacheinander bei 35 bis 50°C 14 g Schwefelsäure (100 gew.-%ig), 27 g wasserfreie Blausäure und 44 g Acetaldehyd (100 gew.-%ig) gegeben. Man rührte solange, bis bei der Titration des Blausäuregehaltes keine Änderung mehr festzustellen war. Nach Abkühlen auf 10°C wurde der Niederschlag abfiltriert und getrocknet. Es resultierten 123,4 g α-D,L-Alaninnitril-N,N-diacetonitril (entsprechend 83 % der Theorie) vom Schmelzpunkt 82°C.

Das erhaltene α-D,L-Alaninnitril-N,N-diacetonitril wurde bei 50°C in 440 g 25 gew.-%iger wäßriger Natronlauge eingetragen, es wurde anschließend noch 2 Stunden bei dieser Temperatur nachgerührt. Danach wurde für 10 Stunden auf 95°C erwärmt. Gegen Ende der Umsetzung wurde das Reaktionsgemisch mit Wasser verdünnt. Man erhielt so 610 g einer wäßrigen Lösung von α-D,L-Alanin-N,N-diessigsäure-Trinatriumsalz mit einem Eisen-Bindevermögen von 1,285 mmol/g (entsprechend 94 % der Theorie, bez. auf eingesetztes α-D,L-Alaninnitril-N,N-diacetonitril).

### Beispiel 2

### Herstellung von α-D,L-Alanin-N,N-diessigsäure-Trinatriumsalz aus α-D,L-Alanin

Zu einer Suspension von 44 g D,L-Alanin (> 99 gew.-%ig) in 200 g Wasser gab man bei 30°C gleichzeitig 105 g Formaldehyd (30 gew.-%ig) und 31,7 g Blausäure (89,5 gew.-%ig). Es wurde noch 3 Stunden bei 30°C nachgerührt. Die Blausäure-Abnahme entsprach einem Umsatz von > 97 % der Theorie.

Die so erhaltene wäßrige Lösung von α-D,L-Alanin-N,N-diacetonitril wurde bei 30°C zu 132 g 50 gew.-%iger Natronlauge getropft. Nach 8-stündigem Rühren bei 30°C wurde die Temperatur auf 95 bis 102°C erhöht. Nach weiteren 4 Stunden war die Umsetzung praktisch vollständig. Man erhielt 352,5 g einer Lösung, die gemäß ihrem Eisen-Bindevermögen 37,4 Gew.-% α-D,L-Alanin-N,N-diessigsäure-Trinatriumsalz enthielt (entsprechend einer Ausbeute von 97,4 % der Theorie über beide Stufen).

### Anwendungstechnische Daten und Anwendungsbeispiele

### Bestimmung des Calcium-Bindevermögens

### Meßprinzip

Die inhibierende Wirkung von Komplexbildnern oder Dispergiermitteln auf die Ausfällung von Calciumcarbonat wird durch Trübungstitration bestimmt. Es wird die zu untersuchende Substanz vorgelegt und in Gegenwart von Natriumcarbonat mit Calciumacetatlösung titriert. Der Endpunkt wird durch Bildung des Calciumcarbonat-Niederschlages angezeigt. Durch Verwendung einer ausreichenden Menge an Natriumcarbonat wird sichergestellt, daß die Messung auch dann ein korrektes Ergebnis liefert, wenn die Wirkung nicht nur auf einer Komplexierung der Calciumionen beruht, sondern auf der Dispergierung von Calciumcarbonat. Werden nämlich zu kleine Natriumcarbonatmengen eingesetzt, besteht die Gefahr, daß das Dispergiervermögen des Produktes nicht ausgeschöpft wird; in diesem Fall wird der Titrationsendpunkt durch die Fällung des Calcium-Salzes der untersuchten Verbindung bestimmt.

Während der Titration wird die Änderung der Lichtdurchlässigkeit mit Hilfe eines Lichtleiterphotometers verfolgt. Bei letzterem wird ein über Glasfaser in die Lösung geleiteter Lichtstrahl an einem Spiegel reflektiert und die Intensität des reflektierenden Lichts gemessen.

### Reagenzien

0,25 m Ca(OAc)₂-Lösung

10 gew.-%ige Na₂CO₃-Lösung

1 n NaOH-Lösung

1 gew.-%ige Salzsäure

### Durchführung

1 g Wirksubstanz (W.S.) in Form des Trinatriumsalzes wird in 100 ml destilliertem H₂O gelöst. Anschließend werden 10 ml 10 gew.%ige Na₂CO₃-Lösung zugegeben. Bei Raumtemperatur (RT) und einem während der Titration konstant gehaltenen pH-Wert von 11 und bei 80°C mit einem pH-Wert von 10 wird mit 0,25 m Ca(OAc)₂-Lösung kontinuierlich mit 0,2 ml/min automatisch titriert.

### Berechnung

Menge mg CaCO₃/g W.S. = Verbrauch an Ca(OAc)₂-Lösung in ml x 25. Bei der automatischen Titration ist der 1. Knickpunkt der Titrationskurve der Endpunkt.

Die folgende Tabelle 1 zeigt die Ergebnisse der Bestimmungen.

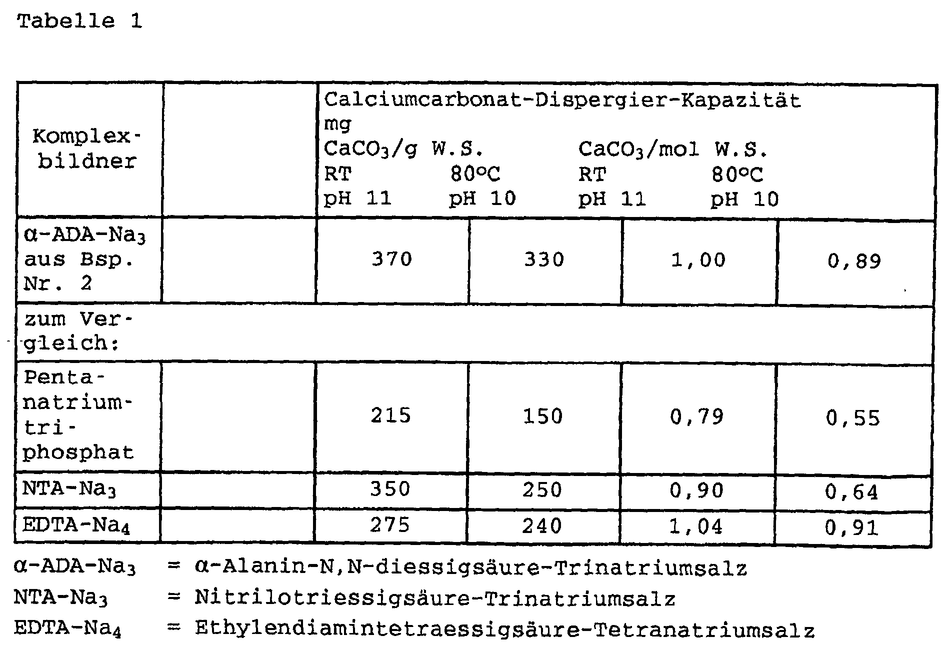

### Beispiel 3

### Hochalkalische Reinigungsmittelformulierung für Molkereien

Eine Mischung aus

| | |
|---|---|
| 40 Gew.-Teilen | 50 gew.-%iger Natronlauge, |
| 20 Gew.-Teilen | einer 30 gew.-%igen wäßrigen Lösung von α-D,L-Alanin-N,N-diessigsäure-Trinatriumsalz aus Bsp. Nr. 2, |
| 4 Gew.-Teilen | eines C₁₀-Oxoalkoholethoxylates mit einem Ethoxylierungsgrad von ca. 4, |
| 4 Gew.-Teilen | einer handelsüblichen Alkylcarbonsäure-Mischung als Lösevermittler, |
| 7 Gew.-Teilen | Natriumgluconat zum Abbau der Wasserhärte und |
| 25 Gew.-Teilen | Wasser |

wurde bei der Entfernung von Ablagerungen aus Calciumphosphat, Calciumoxalat, Eiweiß und Asche eingesetzt. Die Ablagerungen ließen sich problemlos entfernen.

### Beispiel 4

### Hochalkalische Reinigungsmittelformulierung für Brauereien

Eine Mischung aus

| | |
|---|---|
| 40 Gew.-Teilen | 50 gew.-%iger Kalilauge, |
| 20 Gew.-Teilen | einer 30 gew.-%igen wäßrigen Lösung von α-D,L-Alanin-N,N-diessigsäure-Trinatriumsalz aus Bsp. Nr. 2, |
| 3 Gew.-Teilen | eines C₁₀-Oxoalkoholethoxylates mit einem Ethoxylierungsgrad von ca. 3, |
| 3 Gew.-Teilen | einer handelsüblichen Alkylcarbonsäure-Mischung als Lösevermittler und |
| 34 Gew.-Teilen | Wasser |

wurde bei der Entfernung von Ablagerungen aus Calciumoxalat, Hopfenharzen und Eiweiß eingesetzt. Die Ablagerungen ließen sich problemlos entfernen.

## Patentansprüche

1. Verwendung von α-Alanin-N,N-diessigsäure und ihren Salzen der Formel I 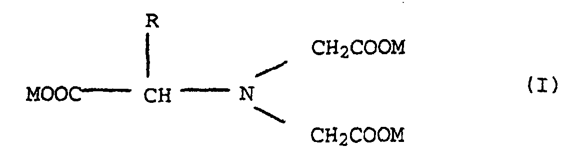 in der
R für Methyl steht und
M Wasserstoff, Alkalimetall, Ammonium oder substituiertes Ammonium bedeutet,
als Komplexbildner für Erdalkali- und Schwermetallionen in alkalischen Reinigungsmittelformulierungen für Reinigungsprozesse in der Getränke- und Nahrungsmittelindustrie.

2. Alkalische Reinigungsmittelformulierungen für die Getränkeund Nahrungsmittelindustrie, enthaltend wirksame Mengen an α-Alanin-N,N-diessigsäure oder ihren Salzen I gemäß Anspruch 1.

3. Alkalische Reinigungsmittelformulierungen nach Anspruch 2 für die Flaschenreinigung in der Getränkeindustrie.

4. Alkalische Reinigungsmittelformulierungen nach Anspruch 2 für die Apparatereinigung in Molkereien, in Brauereien, in der Konservenindustrie, in der Backwarenindustrie, in der Zuckerindustrie, in der fettverarbeitenden Industrie und in der fleischverarbeitenden Industrie.

5. Wäßrige Reinigungsmittelformulierung für die Getränke- und Nahrungsmittelindustrie, enthaltend
(i) 0,05 bis 30 Gew.-% an α-Alanin-N,N-diessigsäure oder ihren Salzen I gemäß Anspruch 1,
(ii) 2 bis 50 Gew.-% Alkalimetallhydroxid und
(iii) 1 bis 30 Gew.-% Tenside.

## Claims

1. The use of α-alanine-N,N-diacetic acid and its salts of the formula I 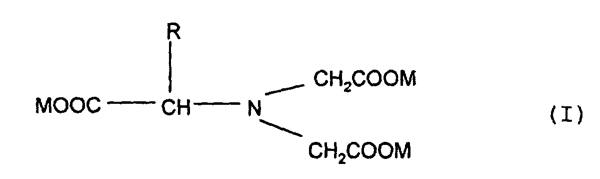 in which
R is methyl, and
M is hydrogen, alkali metal, ammonium or substituted ammonium,
as complexing agents for alkaline earth and heavy metal ions in alkaline cleaner formulations for cleaning processes in the drinks and foodstuffs industries.

2. An alkaline cleaner formulation for the drinks and foodstuffs industries, comprising effective amounts of α-alanine-N,N-diacetic acid or its salts I as claimed in claim 1.

3. An alkaline cleaner formulation as claimed in claim 2 for bottle cleaning in the drinks industry.

4. An alkaline cleaner formulation as claimed in claim 2 for apparatus cleaning in dairies, in breweries, in the conserves industry, in the baked products industry, in the sugar industry, in the fat-processing industry and in the meat-processing industry.

5. An aqueous cleaner formulation for the drinks and foodstuffs industries, comprising
(i) 0.05 to 30% by weight of α-alanine-N,N-diacetic acid or its salts I as claimed in claim 1,
(ii) 2 to 50% by weight of alkali metal hydroxide and
(iii) 1 to 30% by weight of surfactants.

## Revendications

1. Utilisation d'acide α-alanine-N,N-diacétique et de ses sels de formule I 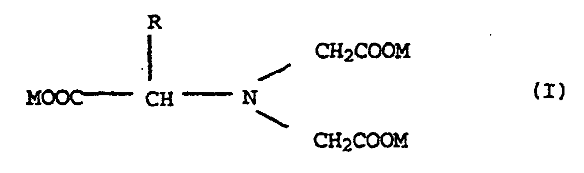 dans laquelle
R représente un groupement méthyle, et
M est un atome d'hydrogène, un atome d'un métal alcalin, un groupement ammonium ou ammonium substitué,
en tant qu'agent complexant pour des ions de métaux alcalino-terreux et de métaux lourds dans des formulations alcalines d'agent nettoyant pour des opérations de nettoyage dans l'industrie des boissons et des produits alimentaires.

2. Formulations alcalines de produits nettoyants pour l'industrie des boissons et des produits alimentaires, contenant des quantités efficaces d'acide α-alanine-N,N-diacétique ou de sels I selon la revendication 1.

3. Formulations alcalines de produits nettoyants selon la revendication 2, pour le nettoyage des bouteilles dans l'industrie des boissons.

4. Formulations alcalines de produits nettoyants selon la revendication 2, pour le nettoyage des appareils dans les laiteries, les brasseries, la conserverie, la biscuiterie, dans l'industrie du sucre, dans l'industrie de transformation des graisses, et dans l'industrie de transformation de la viande.

5. Formulation aqueuse de produits nettoyants pour l'industrie des boissons et des produits alimentaires, contenant
(i) de 0,05 à 30 % en poids d'acide α-alanine-N,N-diacétique ou de ses sels I selon la revendication 1,
(ii) de 2 à 50 % en poids de l'hydroxyde d'un métal alcalin, et
(iii) de 1 à 30 % en poids de tensioactifs.
